# EUROPEAN PATENT APPLICATION

(11) **EP 4 793 353 A1**
(43) Date of publication of application: **19.08.2026**
(21) Application number: 25305200.5
(22) Date of filing: 13.02.2025
(51) Int. Cl.: C12N 15/09, C12N 15/82

(54) **METHOD FOR INCREASING HEAT-STRESS-TOLERANCE OF POLLEN GRAINS**

(71) Applicant: Plantik Biosciences, 45006 Orleans (FR)
(72) Inventor: NAYAK, Aditya P., 45006 ORLEANS (FR); DESTAILLEUR, Alice, 45006 ORLEANS (FR)
(74) Representative: Cabinet Beau de Loménie

(57) **Abstract**

The present invention relates to a method for increasing heat-stress-tolerance of pollen grains of a plant, a method for producing heat-stress-tolerant pollen grains and a heat-stress-tolerant pollen grain.

## Description

### Field of the invention

The present invention relates to a method for increasing pollen grains heat-stress-tolerance of pollen grains in a plant comprising inactivating a BZR1 binding E-Box motif on the Phytochrome-Interacting Factor 4 (PIF4) promoter in the plant, thereby the pollen grain viability is preserved under heat stress.

### Background of the invention

Tomato (*Solanum lycopersicum*) is a globally significant crop, both economically and nutritionally. As a staple in diets worldwide, the tomato is a valuable source of vitamins, antioxidants, and fiber. Despite its adaptability to different climates, tomato production faces increasing challenges due to abiotic stressors, particularly heat stress (HS). High temperatures are detrimental to the plant's reproductive cycle, significantly reducing pollen viability, fruit set, and, consequently, overall yield. The need for tomato varieties capable of maintaining productivity under such harsh environmental conditions is becoming increasingly urgent in the face of rising global temperatures.

Elevated temperatures, especially those exceeding 26°C during the day and 20°C at night, negatively affect tomato plant physiology at multiple levels. At the reproductive stage, heat stress disrupts key processes such as pollen germination and pollen tube development, leading to poor fertilisation and a subsequent reduction in fruit set (PRESSMAN et al., 2002). Additionally, heat stress can trigger flower abscission, thereby limiting the number of fruits produced. These physiological effects not only impact yield but also reduce fruit quality, making it a significant concern for commercial tomato growers. Beyond its reproductive effects, heat stress also affects vegetative growth by impairing photosynthesis, destabilizing cell membranes, and leading to oxidative stress due to the overproduction of reactive oxygen species (ROS). All these factors highlight the urgent need for thermotolerant tomato varieties that can withstand elevated temperatures without compromising yield or quality.

The molecular basis of heat tolerance in plants is governed by a complex network of transcription factors(Kan et al., 2023) and hormonal pathways that regulate responses to environmental stresses. Among these, the interactions between Phytochrome-Interacting Factor 4 (PIF4) and BRASSINAZOLE-RESISTANT 1 (BZR1) have been identified as critical components in the heat stress response. PIF4, a basic helix-loop-helix (bHLH) transcription factor, is essential for the regulation of thermomorphogenesis, the process by which plants adjust their growth patterns in response to elevated temperatures. BZR1, on the other hand, is a key transcription factor in the brassinosteroid (BR) signaling pathway, which regulates a wide array of growth and stress responses. Together, PIF4 and BZR1 coordinate gene expression in response to heat stress, modulating plant growth and ensuring cellular protection (Ding et al., 2020).

Brassinosteroids are plant steroid hormones that play a crucial role in plant growth, development, and stress responses, including thermotolerance. Under heat stress conditions, BRs enhance the activation of thermal protective pathways by stabilising PIF4 and promoting its interaction with BZR1. This interaction induces the expression of heat-responsive genes, many of which contribute to cellular protection by producing heat shock proteins (HSPs) and other stress-related molecules. While this interaction generally enhances a plant's ability to withstand high temperatures, excessive upregulation of PIF4 can have negative consequences. Overexpression of PIF4 under sustained heat stress conditions has been shown to reduce pollen viability, leading to poor fruit set. This phenomenon underscores the importance of precisely regulating PIF4 activity to ensure that thermotolerance is achieved without detrimental side effects, such as impaired reproductive success.

In addition to its role in thermomorphogenesis, PIF4 is involved in various growth and development processes that are influenced by environmental signals. The transcriptional regulation of PIF4 is highly complex, involving interactions with multiple other transcription factors and feedback loops that finely tune its expression and activity. Among these regulators are BZR1, HY5, and MYC2, which together modulate PIF4 in response to environmental cues such as light and temperature. BZR1, acting as part of the brassinosteroid signaling pathway, not only interacts with PIF4 but also directly regulates its expression. This interaction forms a positive feedback loop in which the activation of PIF4 by BZR1 further enhances BZR1's activity, thereby promoting the expression of BR-related genes that confer thermotolerance. While this feedback loop is essential for enabling plants to adapt to heat stress, it can become problematic if PIF4 is excessively upregulated, leading to potential growth defects under prolonged high-temperature conditions.

Recent research, including analyses of multiple chromatin immunoprecipitation sequencing (ChIP-seq) datasets, has identified at least 34 transcription factors that regulate PIF4 expression in Arabidopsis thaliana (Koene et al., 2023; Kulkarni et al., 2018). These findings reveal the complexity of PIF4 regulation and suggest that a multifaceted approach is required to modulate its activity effectively under heat stress conditions. This complexity also highlights the risks of indiscriminately modifying PIF4 expression. For example, experiments involving the constitutive silencing of SIPIF4, the tomato homolog of AtPIF4 (Arabidopsis thaliana PIF4), using RNA interference (RNAi) techniques, have shown that while reducing PIF4 levels can mitigate heat stress symptoms, it also results in pleiotropic effects. Silencing SIPIF4 leads to reduced vegetative growth, fewer flowers, and smaller fruit size under normal growth conditions (Rosado et al., 2019). This underscores the necessity of a targeted approach that specifically addresses PIF4 overexpression under heat stress, without causing undesirable side effects under normal temperatures.

To address these challenges, the present invention provides a novel solution for enhancing heat tolerance in tomato plants. The invention proposes targeting and attenuating the heat-induced overexpression of the SlbHLH9 transcription factor (also known as PIF4) by inactivating a specific regulatory element within its promoter: the E-box motif. The E-box is a conserved DNA sequence recognized by bHLH transcription factors, including bHLH9(PIF4) and BZR1, and has been shown to play crucial roles in regulating gene expression in response to heat stress in *Arabidopsis thaliana* (Ibañez et al., 2018). By specifically inactivating this E-box motif in tomatoes, the invention aims to modulate the expression of heat stress-responsive genes, thereby improving the plant's ability to maintain pollen viability and fruit set under elevated temperatures.

This targeted genetic editing provides a robust and precise method for enhancing thermotolerance in tomato plants. As shown here, the inactivation of the E-box motif in the promoter of SIPIF4 suppresses heat-sensitive pathways that contribute to pollen sterility, while simultaneously enhancing the activation of protective genes involved in stress tolerance. As a result, the tomato plants engineered using this approach thrive under elevated temperature conditions, maintaining high productivity without sacrificing fruit quality.

The present invention represents a significant advancement in the development of heat-tolerant tomato varieties. By selectively manipulating the transcriptional activity of bHLH9 through inactivation of the E-box motif, the invention offers a novel and targeted solution to the ongoing challenges posed by climate change and increasing global temperatures. Furthermore, the applicability of this approach extends beyond tomatoes, as it can be used in other members of the Solanaceae family, or even other plant families, providing a versatile tool for improving crop resilience in heat-stressed environments. This innovation has the potential to significantly impact global food security by enabling the cultivation of heat-tolerant crop varieties in regions where rising temperatures threaten agricultural productivity.

### Summary of the invention

In a first aspect, the present invention provides a method for increasing heat-stress-tolerance of pollen grains of a plant, comprising inactivating a E-Box motif of the Phytochrome-Interacting Factor 4 (PIF4) promoter in the plant, wherein said E-Box motif has a nucleotide sequence of GACGTC.

In a second aspect, the present invention provides a method for producing heat-stress-tolerant pollen grains, said method comprising the steps of:
(i) providing a plant in which a E-Box motif of the PIF4 promoter having a nucleotide sequence of GACGTC is inactivated;
(ii) culturing the plant under appropriate conditions to produce pollen grains, thereby obtaining heat-stress-tolerant pollen grains.

In a third aspect, the present invention provides a heat-stress-tolerant pollen grain produced by a plant in which a E-Box motif of the PIF4 promoter having a nucleotide sequence of GACGTC is inactivated.

In some embodiments, the E-Box motif is inactivated by introducing one or more mutations in the nucleotide sequence GACGTC.

In some embodiments, the E-Box motif is inactivated by introducing one or more mutations in the nucleotides sequence ACGT of the nucleotide sequence GACGTC.

In some embodiments, the one or more mutation is selected from the group consisting of an insertion, a deletion, a substitution and combinations thereof.

In some embodiments, the E-Box motif is inactivated by deleting a nucleotide sequence comprising the nucleotide sequence ACGT of the nucleotide sequence GACGTC.

In some embodiments, the PIF4 promoter has the sequence SEQ ID NO: 5.

In some embodiments, the E-Box motif is inactivated by deleting the nucleotide sequence SEQ ID NO: 1 or SEQ ID NO: 3.

In some embodiments, the PIF4 promoter in the plant has two E-Box motifs, such as the E-Box motif having the nucleotide sequence of GACGTC and a E-Box motif having the nucleotide sequence of CACGTT.

In some embodiments, a E-Box motif of the PIF4 promoter in the plant having a nucleotide sequence of CACGTT is not inactivated.

In some embodiments, the PIF4 promoter in the plant in which the E-Box motif of GACGTC is inactivated comprises the nucleotide sequence SEQ ID NO: 2 or SEQ ID NO: 4.

In some embodiments, the plant belongs to the spermatophyte clade.

In some embodiments, the plant belongs to the angiosperm clade or gymnosperm clade.

In some embodiments, the plant belongs to the *Solanaceae* family.

In some embodiments, the plant belongs to the *Solanum* genus.

In some embodiments, the plant belongs to the species *Solanum lycopersicum.*

The details of one or more embodiments of the present invention are set forth in the description below. Other features, objects and advantages of the invention will be apparent from the description.

### Brief description of the figures

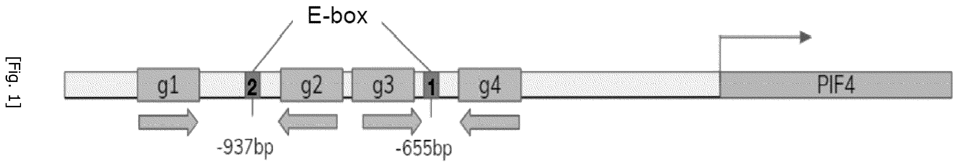

[Fig. 1] Representation of the PIF4 gene and its promoter sequence. E-Box motif 1 (i.e., GACGTC) is located at -655 base pairs from the PIF4 gene. E-Box motif 2 (i.e., CACGTT) is located at -937 base pairs from the PIF4 gene. The guides used for the deletion of the E-Boxes are also represented (arrows).

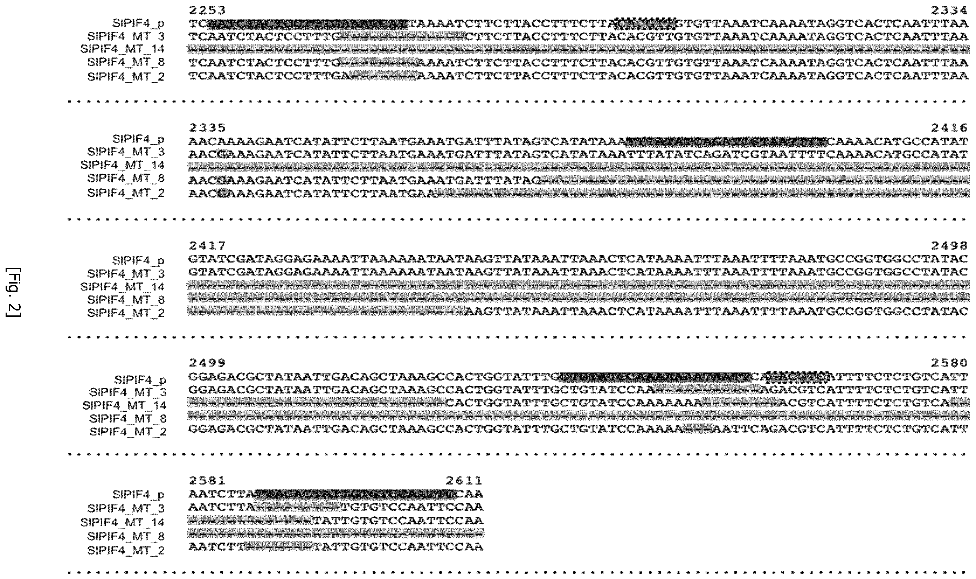

[Fig. 2] PIF4 promoter nucleotide sequences of homozygous tomato mutants having different deletions in the nucleotide sequence of the PIF4 promoter. Bases underlined in blue correspond to the guides position and bases underlined in green represent the E-Box motif. SIPIF4 mutant corresponds to the unmodified sequence of the PIF4 promoter. SIPIF4_MT_3 mutant comprises deletions but intact E-Box motifs. SIPIF4_MT_14 mutant comprises a deletion of the E-Box motif 2. SIPIF4_MT_8 mutant comprises a deletion of E-Box motif 1 (mutant according to the invention). SIPIF4_MT_2 mutant comprises a deletion of the area in between the E-Box motif 1 and the E-Box motif 2.

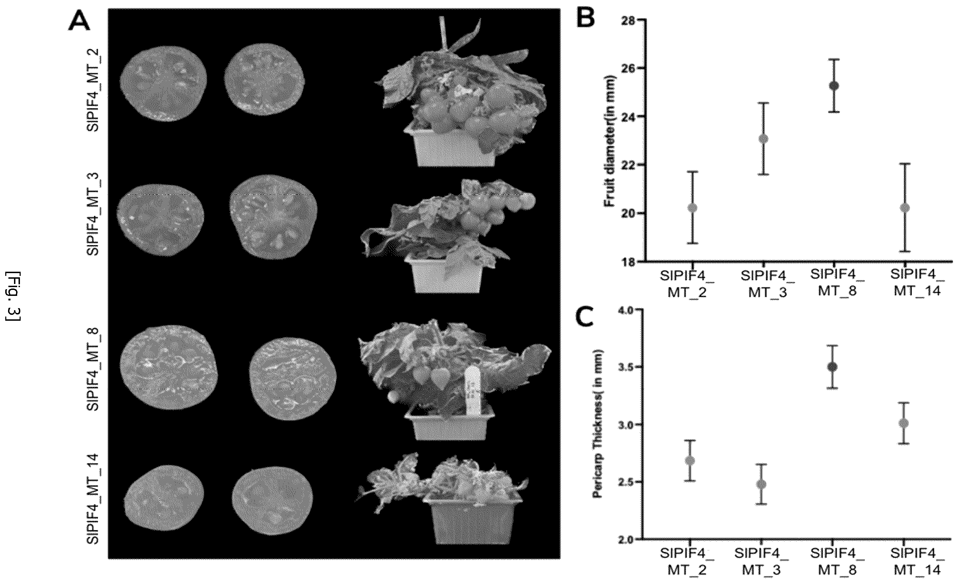

[Fig. 3] E-Box motif deletions are associated with various types of pericarp thickness phenotypes. A) Pictures of half fruits from the second truss and the corresponding full plants. B) Measures of the fruit diameter (mm). C) Measures of the pericarp thickness (mm). Figures B and C represents the mean with 95% confidence interval (n=>10).

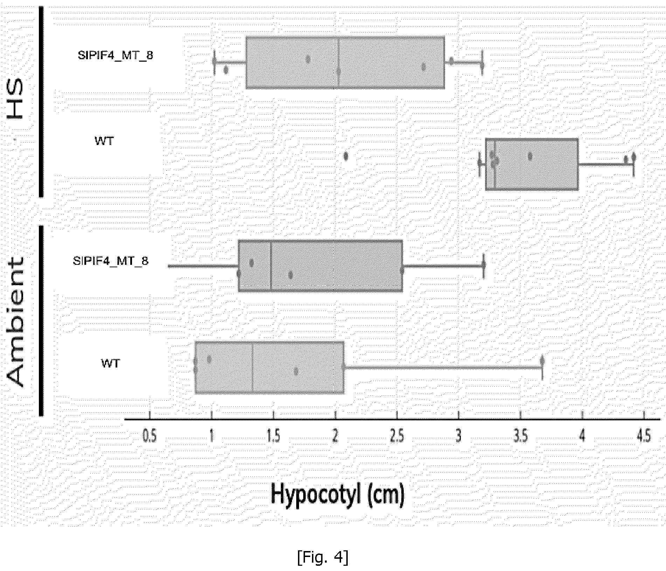

[Fig. 4] E-Box motif deletion is associated with an increase in heat-stress tolerance. Hypocotyl elongation (in cm) of WT and PIF4_8 mutants in normal (16h/8h - 25°C/18°C, named "Ambient") and heat stress conditions (16h/8h - 30°C/30°C - 7 days, named "HS").

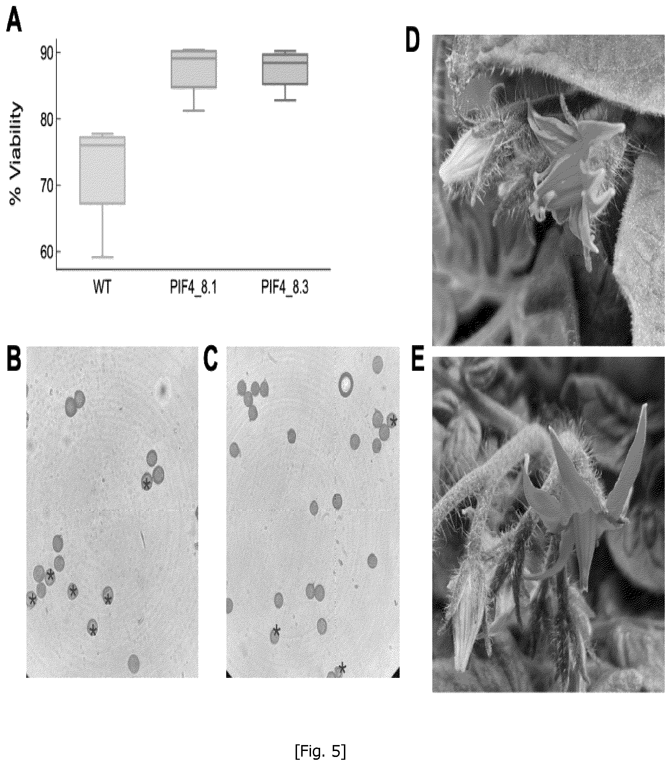

[Fig. 5] E-Box motif deletion increases pollen grains viability under heat stress. A) Pollen grains viability of WT and SIPIF4_MT. B) Alexander staining of WT pollen grain after 2 hours under heat stress conditions (37°C). C) Alexander staining of SIPIF4_MT_8 mutant pollen grains after 2 hours in heat stress conditions (37°C). D) WT flower developed under heat stress condition (38°C day 16h/28°C night 8h). E) SIPIF4_MT_8 mutant flowers developed under heat stress conditions (38°C day 16h/28°C night 8h). Black stars in Figures B and C indicate dead pollen grains.

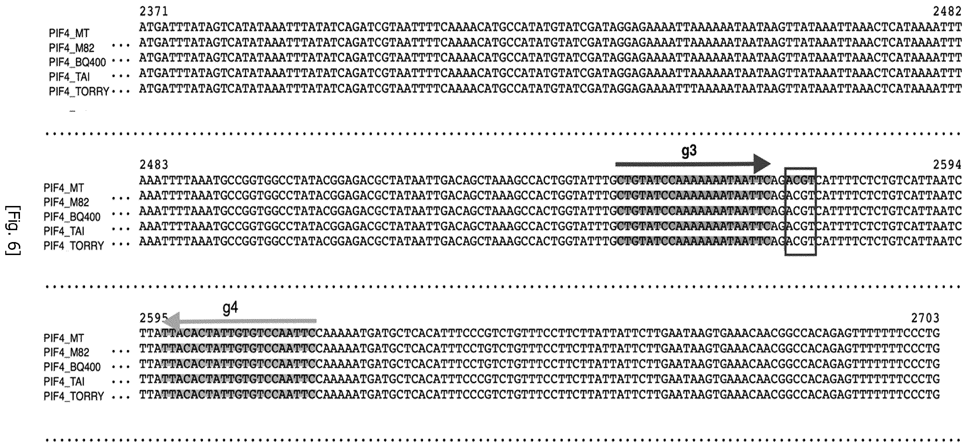

[Fig. 6] E-box motif from SIPIF4 promoter is conserved between tomato genotypes. Alignment of SIPIF4 promoter sequence from -542 to -875 bp upstream the start codon showing the perfect conservation of guides g3 and g4 and E-box motif between the tomato genotypes MicroTom (MT), M82 and the tree hybrids commercial lines BQ400, TAI and TORRY. Guides position and highlighted in gray and E-Box motif is framed.

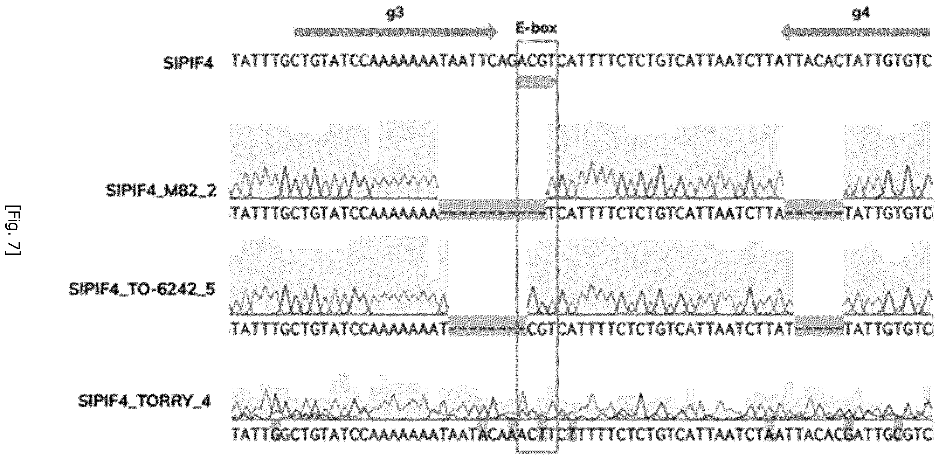

[Fig. 7] PIF4 promoter nucleotide sequences of homozygous and heterozygous tomato mutants having different deletions in the nucleotide sequence of the PIF4 promoter. G3 and g4 guide positions are indicated by an arrow and E-box core motif position is framed. Deleted base pairs are indicated by a dash. SIPIF4 corresponds to the unmodified sequence of the PIF4 promoter. SIPIF4_M82_2 mutant comprises homozygous deletions of 3 of the 4 base pairs of the E-Box motif 1. SIPIF4_TO-6242_5 mutant comprises homozygous deletions of 1 of the 4 base pairs of the E-Box motif 1. SIPIF4_TORRY_4 mutant comprises heterozygous deletion between g3 and g4.

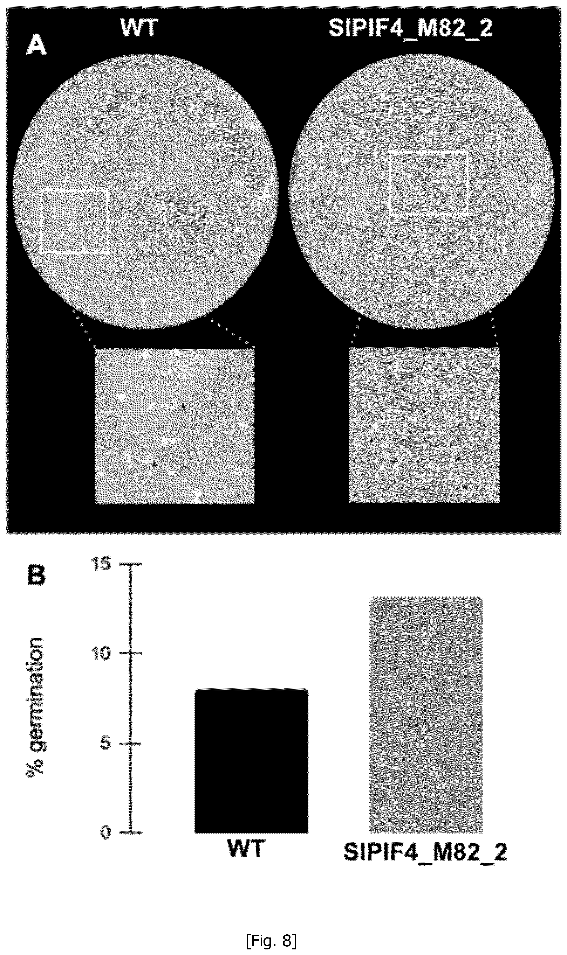

[Fig. 8] E-Box motif 1 deletion in SIPIF4 promoter is associated with an increase of pollen germination efficiency under heat stress conditions in SIPIF4_M82_2 mutant. A) and B) show pollen grain germination efficiency test under heat stress conditions (16h - 37°C) for WT and SIPIF4_M82_2 lines. A) Image of pollen of the germination medium after 16 hours of incubation acquired with a binocular magnifier. Black stars indicate germinated pollen grains. B) Percentage of germinated pollen grains calculated as the total number of germinated pollen grains/total number of pollen grains.

### Detailed description

### Definitions

The term "plant" includes reference to a whole plant, a part of a plant, a grafted plant and progeny of the plants, including seeds, shoots, stems, roots (including tubers), rootstock, scion, plant cells, tissues, and organs. The plant may be in any form including suspension cultures, embryos, meristematic regions, callus tissue, leaves, gametophytes, sporophytes, pollen, and microspores. The plant according to the invention can produce pollen grains when it is cultured under appropriate conditions.

The term "progeny" comprises any subsequent generation of a plant. A "T0 plant" is a parental plant that has been recovered from a transformation and regeneration process. Progeny of T0 plants is referred to as T1 (first progeny generation), T2 (second progeny generation), etc.

The term "spermatophyte" or "seed plant" refers to any plant that produces seeds.

The terms "*Gymnospermae"* and "Gymnosperms" refer to plants that produce seeds, but not flowers and fruits. Gymnosperms include conifers, cycads, Ginkgo, and gnetophytes. *Gymnospermae* plants include *Pinopsida, Cycadopsida, Ginkgoopsida,* and *Gnetopsida* plants.

The terms "*Angiospermae"* and "Angiosperms" refer to plants that produce flowers and fruits. *Angiospermae* include *Magnoliopsida, Liliopsida, Rosopsida, Asteropsida, Caryophyllidae, Hamamelidiaceae,* and *Magnoliidae.*

The term "*Solanaceae"* refers to a plant family of flowering plants that ranges from annual and perennial herbs to vines, lianas, epiphytes, shrubs, and trees, and includes several agricultural crops, medicinal plants, spices, weeds, and ornamentals.

The term "*Solanum"* refers to a large genus of flowering plants, which include three food crops of high economic importance: the potato plant, the tomato plant and the eggplant. It also contains the so-called horse nettles, as well as several plants cultivated for their ornamental flowers and fruit.

The term *"Solanum lycopersicum",* also known as *Lycopersicon esculentum,* refers to the tomato plant.

The term "wild type plant" or "wt plant" refers to the phenotype of the typical form of a plant as it generally occurs in nature or results from breeding under natural conditions. A "wild type" is conceptualized as a plant of the standard normal or more frequent phenotype, in contrast to phenotypes that may result from mutation.

The term "control plant" provides a reference point for measuring changes in phenotype of a subject plant, such as a subject pollen grains, in which genetic modification has been affected as to a sequence of interest.

The term "phenotype" as used herein means the detectable characteristics of a cell or organism such as plants.

The term "mutation" refers to a change in the nucleotide sequence. Such mutation includes at least one substitution, deletion, and/or insertion in the nucleotide sequence. By "substitution" is meant the replacement of at least one nucleotide in a nucleotide sequence with at least one other nucleotide. By "insertion" is meant the addition of at least one nucleotide in a nucleotide sequence. By "deletion" is meant the removal of at least one nucleotide in a nucleotide sequence.

The terms "promoter" and "transcriptional promoter" are equivalent and refer to a control region of a nucleic acid sequence at which transcription initiation and rate of transcription of the remainder of a nucleic acid sequence are controlled. A promoter may also contain subregions to which regulatory proteins and molecules may bind, such as RNA polymerase and other transcription factors. A promoter drives transcription of the nucleic acid sequence that it regulates. Herein, a promoter is "operably linked" when it is in a correct functional location and orientation in relation to a nucleic acid sequence it regulates to control ("drive") transcriptional initiation of that sequence. A "plant promoter" is a promoter capable of initiating transcription in plant cells.

The term "Phytochrome-Interacting Factor 4" or "PIF4" refers to a basic-helix-loop-helix (bHLH) transcription factor that functions as a negative regulator of phytochrome B signalling. PIF4 is known to be well conserved in the *Plantae* kingdom. Alternative names of PIF4 are Basic helix-loop-helix protein 9 (short name: AtbHLH9 or bHLH 9), Phytochrome-interacting factor 4, Short under red-light 2 (SRL2), Transcription factor EN102 and bHLH transcription factor (bHLH009). PIF4 is for example described in databases under the following NCBI ID numbers: Arabidopsis thaliana: 818903; Oryza sativa (Riz): 4342383; Zea mays (maïs): 100382453; Solanum Lycopiersicum (tomate): 101252303; Capsicum annuum (poivron): 107878077; Solanum tuberosum (pomme de terre): 102604886. PIF4 protein has been shown to bind the regulatory E-box and E-Box motifs of its own promoter. Such E-Box motif can be repeated in the PIF4 promoter.

The term "E-Box motif" refers to a highly conserved DNA sequence that has been identified in the 5' upstream region of plant genes exhibiting regulation by a variety of environmental signals and physiological cues. Preferably, the term "E-Box motif" refers to a genetic signature which comprises a core sequence of a E-Box motif, more preferably comprised in the PIF4 promoter.

The term "plasmid" or "vector" refers to a nucleic acid molecule that serves to transfer a passenger nucleic acid sequence, such as DNA or RNA, into a host cell. A vector may comprise an origin of replication, a selectable marker, and optionally a suitable site for the insertion of a sequence or gene. A vector can be either a self-replicating extrachromosomal vector or a vector which integrates into a host genome. It can also comprise expression elements including, for example, a promoter, the correct translation initiation sequence such as a ribosomal binding site and a start codon, a termination codon, and a transcription termination sequence. A nucleic acid construct may also comprise other regulatory regions such as enhancers, silencers and boundary elements/insulators to direct the level of transcription of a given gene. There are several common types of vectors including nucleic acid constructs, bacterial virus genomes, phagemids, virus genomes, cosmids, and artificial chromosomes. The nucleic acid construct can be a vector for stable or transient expression of a gene or sequence.

The term "inactivation" refers to the direct or indirect inhibition or decrease of the expression of the biological function of a regulatory element, gene or protein, compared to a normal or previous condition. The regulation of the genetic element can be on itself (i.e., cleavage, modifications), at the stage of transcription (i.e., using silencers or repressors), or using RNAi (e.g., siRNA, shRNA, endogenous microRNA or artificial microRNA), TALEN, Zinc Finger (ZFN), meganuclease, or CRISPR/Cas strategy.

The term "inactivation of the E-Box motif" refers to the decrease or the inhibition of the binding of transcription factor(s) on this particular motif. This means that when the E-Box motif is inactivated, the transcription factor(s) cannot recognize nor bind the E-Box motif of the PIF4 promoter due to its mutation, preferably deletion. In a particular aspect, the inactivation of the E-Box motif refers to the reduction or inhibition of the binding of the BZR1 protein on the E-Box motif of the PIF4 promoter.

The term "introduced" in the context of inserting a construct into a cell, means "transfection" or "transformation" or "transduction" and includes reference to the incorporation of a nucleic acid fragment into a cell (e.g., *Solanum lycopersicum*), where the nucleic acid fragment may be incorporated into the genome of the cell, converted into an autonomous replicon, or transiently expressed.

The term "transformation" as used herein refers to both stable transformation and transient transformation. "Stable transformation" refers to the introduction of a nucleic acid fragment into a genome of a host organism resulting in genetically stable inheritance. Once stably transformed, the nucleic acid fragment is stably integrated in the genome of the host organism and any subsequent generation. "Transient transformation" refers to the introduction of a nucleic acid fragment into the nucleus, or DNA-containing organelle, of a host organism resulting in gene expression without genetically stable inheritance. A "transformed cell" is any cell into which a construct has been introduced.

The term "CRISPR-Cas system" refers to Clustered Regularly Interspersed Short Palindromic Repeats-CRIPSR-associated system. The CRISPR-Cas system refers to a tool for generating modifications in genomic DNA at target sites. CRISPR-Cas system may be used to create targeted double-strand or single-strand breaks, and can be used for, without limitation, targeted mutagenesis, gene targeting, gene replacement, targeted deletions, targeted inversions, targeted translocations, targeted insertions, and multiplexed genome modification.

The term "ambient temperature" refers to the air temperature of an environment, particularly in which a plant naturally grows. For example, an ambient temperature may be from 18°C to 25°C, such as 18°C during the night and 25°C during the day.

The terms "increased temperature", "increased ambient temperature", "elevated temperature" or "high temperature" refer to temperature or temperature range that is above the ambient temperature in which a plant naturally grows.

The term "heat stress" refers to a prolonged exposure of a plant, such as pollen grains, to temperatures which are substantially greater than those which are optimal for the same control plant (e.g., wild type). For example, a plant is subjected to heat-stress when the ambient temperatures where the plant is growing are significantly above those normally expected for the plant to naturally grow, for example more than 5°C, such as more than 7°C, more than 8°C, more than 9°C, more than 10°C above temperatures normally expected. Examples of heat stress conditions are daytime temperatures above 30°C, especially above 35°C and night-time temperatures above 20°C, especially above 25°C. Heat stress affects the pollen grains viability in wild-type plant or control plant.

The term "pollen grain" refers to the male gamete produced by a plant.

As used herein, the terms "heat-stress-tolerance" means that the pollen grains show an increased tolerance to increased ambient temperature, compared to a wild type or control plant in the same conditions. Heat-stress-tolerance can be assessed using well known measurement and analysis methods, such as by measuring pollen grains viability.

The term "pollen grain viability" refers to the ability of pollen grains to germinate and fertilize an ovule, leading to seed or fruit development. The pollen grains viability is important for the pollination and subsequent plant reproduction. The pollen grains viability can be measured by assessing, for example, the pollen grains fertility, the pollen grains abortion, and/or the pollen grains death. Examples of methods for measuring the pollen grains death comprise the Alexander staining protocol (Alexander M, 1969), the use of TTC (2,3,5-triphenyl tetrazolium chloride) and IKI (iodine potassium iodide), the use of FDA (fluorescein diacetate).

The term "pollen grains fertility" refers to the reproductive ability of pollen grains by initiating and supporting the fertilization process. Its contact (i.e., fusion) with a female reproductive structure (i.e., an ovule) leads to seed or fruit formation. Thus, the pollen grains fertility is involved in the plant sexual reproduction. The pollen grain fertility may be used proportionally to assess the pollen grains viability. Examples of methods for measuring pollen grains fertility comprise the germination test to evaluate the pollen grains fertility (Karapos et al., 2010).

The term "pollen grains abortion" refers to grains which has not completed the fertilization process, leading to their incapacity to, among others, germinate, grow pollen tubes, and achieve fusion with a female reproductive structure (i.e., an ovule). This leads to the absence of seed or fruit formation. The pollen grains abortion may be used to assess inversely the pollen grains viability. Examples of methods for measuring pollen grains abortion comprise (Karapos et al., 2010).

The term "pollen grains death" refers to the cessation of biological activity of pollen grains with an irreversible loss of functionalities in pollen grains, leading to their incapacity to, among others, germinate, grow pollen tubes, and achieve fusion with a female reproductive structure (i.e., an ovule). This leads to the absence of seed or fruit formation. The pollen grains death may be used to assess inversely the pollen grains viability. Examples of methods for measuring the pollen grains death comprise the Alexander staining protocol (Alexander M, 1969).

### Methods of the invention

The present invention provides a method for increasing heat-stress-tolerance of pollen grains of a plant, comprising inactivating a E-Box motif of the Phytochrome-Interacting Factor 4 (PIF4) promoter in the plant, wherein said E-Box motif has a nucleotide sequence of GACGTC.

The present invention also provides a method for producing heat-stress-tolerant pollen grains, said method comprising the steps of:
(i) providing a plant in which a E-Box motif of the PIF4 promoter having a nucleotide sequence of GACGTC is inactivated;
(ii) culturing the plant under appropriate conditions to produce pollen grains, thereby obtaining heat-stress-tolerant pollen grains.

The Applicant has surprisingly shown that heat-stress-tolerance of pollen grains is increased when said pollen grains are produced by a plant in which a E-Box motif of the PIF4 promoter having a nucleotide sequence of GACGTC is inactivated, as compared to pollen grains produced by a wild-type plant or a control plant. Thus, the pollen grains produced according to the invention have preserved viability under heat-stress, as compared to pollen grains produced by a wild-type plant or a control plant.

In some embodiments, the heat-stress-tolerance of said pollen grains is increased by at least 5%, such as at least 6%, at least 7%, at least 8%, at least 9%, at least 10%, at least 11%, at least 12%, at least 13%, at least 14%, at least 15%, at least 16%, at least 17%, at least 18%, at least 19% or at least 20%, as compared to pollen grains produced by a wild-type plant or a control plant.

It is known in the art that plants are mainly cultured under optimal temperature and conditions to reduce their stress and optimize their growth. Average range of physiological temperature has been described in the art for cereal, horticultural and legume crops (Qunying Luo, 2011). For example, it is known in the art that rice is preferably grown at an ambient temperature between 25°C and 40°C, *Solanum lycopersicum* is preferably grown at an ambient temperature between 18°C and 27°C and *Arabidopsis thaliana* is preferably grown at an ambient temperature between 16°C and 25°C, with an optimal temperature comprised between 22°C and 23°C (The Arabidopsis Biological Resource Center).

In some embodiments, the heat-stress corresponds to an increase in ambient temperature in which the plant preferably grows from 2°C to 10°C, such as from 2°C to 5°C or from 5°C to 10°C, for example an increase in ambient temperature of 2°C, 3°C, 4°C, 5°C, 6°C, 7°C, 8°C, 9°C or 10°C, compared to the ambient temperature in which the plant preferably grows.

For *Solanum lycopersicum,* for example, the heat stress condition may correspond to (i) a daytime temperature above 29°C, such as at least 30°C, at least 31°C, at least 32°C, at least 33°C, at least 34°C, at least 35°C, at least 36°C, at least 37°C, at least 38°C, at least 39°C or at least 40°C, and/or (ii) a night-time temperature above 20°C, such as at least 21°C, at least 22°C, at least 23°C, at least 24°C, at least 25°C, at least 26°C, at least 27°C, at least 28°C, at least 29°C or at least 30°C.

In a specific embodiment, the heat-stress for pollen grains corresponds to an increase in ambient temperature from 2°C to 10°C, such as from 2°C to 5°C or from 5°C to 10°C, for example an increase in ambient temperature of 2°C, 3°C, 4°C, 5°C, 6°C, 7°C, 8°C, 9°C or 10°C, compared to a temperature in which the plant producing the pollen grains preferably grows.

For pollen grains of *Solanum lycopersicum,* for example, the heat stress condition may correspond to a temperature above 29°C, such as at least 30°C, at least 31°C, at least 32°C, at least 33°C, at least 34°C, at least 35°C, at least 36°C, at least 37°C, at least 38°C, at least 39°C or at least 40°C. Another example of heat stress condition may correspond to (i) a daytime temperature above 29°C, such as at least 30°C, at least 31°C, at least 32°C, at least 33°C, at least 34°C, at least 35°C, at least 36°C, at least 37°C, at least 38°C, at least 39°C or at least 40°C, and/or (ii) a night-time temperature above 20°C, such as at least 21°C, at least 22°C, at least 23°C, at least 24°C, at least 25°C, at least 26°C, at least 27°C, at least 28°C, at least 29°C or at least 30°C

In some embodiments, the viability of at least 80%, such as at least 85%, at least 90% or at least 95% of the pollen grains is preserved under a temperature from 2°C to 10°C, such as from 2°C to 5°C or from 5°C to 10°C above the ambient temperature, for example 2°C, 3°C, 4°C, 5°C, 6°C, 7°C, 8°C, 9°C or 10°C above the ambient temperature in which the plant producing the pollen grains preferably grows.

For *Solanum lycopersicum,* for example, the viability of at least 80%, such as at least 85%, at least 90% or at least 95% of the pollen grains may be preserved under (i) daytime temperature above 29°C, such as at least 30°C, at least 31°C, at least 32°C, at least 33°C, at least 34°C, at least 35°C, at least 36°C, at least 37°C, at least 38°C, at least 39°C or at least 40°C and/or (ii) night-time temperature above 20°C, such as at least 21°C, at least 22°C, at least 23°C, at least 24°C, at least 25°C, at least 26°C, at least 27°C, at least 28°C, at least 29°C or at least 30°C. For example, the viability of at least 80%, such as at least 85% of the pollen grains may be preserved under (i) daytime temperature above 35°C, such as 38°C and (ii) night-time temperature above 25°C, such as 28°C.

In another embodiments, the viability of at least 80%, at least 85%, at least 90% or at least 95% of the pollen grains is preserved after 2 hours under heat-stress conditions, preferably after 2 hours at a temperature of at least 35°C, such as at least 36°C, at least 37°C, at least 38°C, at least 39°C or at least 40°C.

For *Solanum lycopersicum,* for example, the viability of at least 80%, such as at least 85%, at least 90% or at least 95% of the pollen grains may be preserved after 2 hours at a temperature of at least 35°C, such as at least 36°C, at least 37°C, at least 38°C, at least 39°C or at least 40°C.

In some embodiments of the invention, the heat-stress does not lead to a decrease of more than 20%, such as of more than 15%, such as of more than 10% or more than 5% in the viability of the pollen grains.

The heat-stress-tolerance of pollen grains can be assessed by measuring pollen grains viability. The pollen grains viability may be measured by assessing the plant pollen grains fertility, the pollen grains abortion and/or the plant pollen grains death. Methods for measuring pollen grains viability are disclosed in the present description.

In some embodiments, the inactivation of the E-Box motif is carried out by introducing mutation in the E-Box, the mutation being selected from the group consisting of an insertion, a deletion, a substitution and combinations thereof.

In some embodiments, the E-Box motif is inactivated by introducing one or more mutations in the nucleotide sequence GACGTC. Preferably, the E-Box motif is inactivated by introducing one or more mutations in the nucleotides sequence ACGT of the nucleotide sequence GACGTC.

For example, the E-Box motif may be inactivated by (i) mutating the nucleotide A of the sequence ACGT in the nucleotide sequence GACGTC; and/or (ii) mutating the nucleotide C of the sequence ACGT in the nucleotide sequence GACGTC; and/or (iii) mutating the nucleotide G of the sequence ACGT in the nucleotide sequence GACGTC ; and/or (iv) mutating the nucleotide T of the sequence ACGT in the nucleotide sequence GACGTC. For example, the E-Box motif may be inactivated by (i) mutating the nucleotides A and C of the sequence ACGT in the nucleotide sequence GACGTC; (vi) mutating the nucleotides C and G of the sequence ACGT in the nucleotide sequence GACGTC; (vii) mutating the nucleotides G and T of the sequence ACGT in the nucleotide sequence GACGTC; (viii) mutating the nucleotides A, C and G of the sequence ACGT in the nucleotide sequence GACGTC; (ix) mutating the nucleotides C, G and T of the sequence ACGT in the nucleotide sequence GACGTC; (x) mutating the nucleotides A, C, G and T of the sequence ACGT in the nucleotide sequence GACGTC.

In some embodiment, the E-Box motif is inactivated by a deletion of one, two, three or four nucleotides in the nucleotides sequence ACGT of the nucleotide sequence GACGTC. A deletion of 2 nucleotides may consist in the deletion of the nucleotides AC, CG or GT of the nucleotide sequence GACGTC. A deletion of 3 nucleotides may consist in the deletion of the nucleotides CGT or ACG of the nucleotide sequence GACGTC. A deletion of 4 nucleotides consists in the deletion of the nucleotides ACGT of the nucleotide sequence GACGTC.

In some embodiments, the E-Box motif is inactivated by deleting a nucleotide sequence which comprises one, two, three or four nucleotides of the nucleotide sequence ACGT of the nucleotide sequence GACGTC. For example, the E-Box motif is inactivated by deleting a nucleotide sequence comprising the nucleotide sequence ACGT of the nucleotide sequence GACGTC. In another example, the E-Box motif is inactivated by deleting a nucleotide sequence comprising the nucleotide sequence ACG of the nucleotide sequence GACGTC. In another example, the E-Box motif is inactivated by deleting a nucleotide sequence comprising the nucleotide sequence AC of the nucleotide sequence GACGTC. In another example, the E-Box motif is inactivated by deleting a nucleotide sequence comprising the nucleotide A of the nucleotide sequence GACGTC. In another example, the E-Box motif is inactivated by deleting a nucleotide sequence comprising the nucleotide sequence CGT of the nucleotide sequence GACGTC. In another example, the E-Box motif is inactivated by deleting a nucleotide sequence comprising the nucleotide sequence GT of the nucleotide sequence GACGTC. In another example, the E-Box motif is inactivated by deleting a nucleotide sequence comprising the nucleotide T of the nucleotide sequence GACGTC. In these embodiments, a nucleotide sequence from 2 nucleotides to 300 nucleotides may be deleted in the PIF4 promoter sequence. Accordingly, the PIF4 promoter of the plant can comprise a deletion of a nucleotide sequence of 300, 290, 280, 270, 260, 250, 240, 230, 220, 210, 200, 190, 180, 170, 160, 150, 140, 130, 120, 110, 100, 90, 80, 70, 60, 50, 40, 30, 20, 10, 9, 8, 7, 6, 5, 4, 3 or 2 bp, such nucleotide sequence comprising one, two, three or four nucleotides of the nucleotides sequence ACGT of the nucleotide sequence GACGTC.

In some specific embodiments, the E-Box motif is inactivated by deleting a nucleotide sequence comprising the nucleotide sequence ACGT of the sequence GACGTC. In these embodiments, a nucleotide sequence from 4 nucleotides to 300 nucleotides may be deleted in the PIF4 promoter sequence, given that a deletion of 4 nucleotides consists in the deletion of the nucleotide sequence ACGT of the nucleotide sequence GACGTC. Accordingly, the PIF4 promoter of the plant can comprise a deletion of a nucleotide sequence of 300, 290, 280, 270, 260, 250, 240, 230, 220, 210, 200, 190, 180, 170, 160, 150, 140, 130, 120, 110, 100, 90, 80, 70, 60, 50, 40, 30, 20, 10, 9, 8, 7, 6, 5 or 4 bp, such nucleotide sequence comprising the nucleotides sequence ACGT of GACGTC.

In some embodiments, the E-Box motif is inactivated by deleting a nucleotide sequence comprising the E-Box motif of GACGTC. In these embodiments, a nucleotide sequence from 6 nucleotides to 300 nucleotides may be deleted in the PIF4 promoter sequence, given that a deletion of 6 nucleotides consists in the deletion of the nucleotide sequence GACGTC. Accordingly, the PIF4 promoter of the plant can comprise a deletion of a nucleotide sequence of 300, 290, 280, 270, 260, 250, 240, 230, 220, 210, 200, 190, 180, 170, 160, 150, 140, 130, 120, 110, 100, 90, 80, 70, 60, 50, 40, 30, 20, 10, 9, 8, 7 or 6 bp, such nucleotide sequence comprising the E-Box motif of GACGTC.

In a specific embodiment, the plant is *Solanum lycopersicum* and the E-Box motif is inactivated by deleting the nucleotide sequence GACGTC.

The E-Box having the nucleotide sequence of GACGTC is in the 5' upstream region of the PIF4 gene (i.e., upstream of the PIF4 gene start codon). In one embodiment, the E-Box motif of GACGTC is within 2 kilobase pair (kbp) upstream of the PIF4 gene start codon, preferably the E-Box motif of GACGTC is within 1 kilobase pair (kbp) upstream of the PIF4 gene start codon.

In one embodiment, the mutation of the E-Box motif of GACGTC is present in the plant in a homozygous state. In another embodiment, the mutation of the E-Box motif of GACGTC is present in the plant in a heterozygous state.

Mutagenesis is commonly used to induce mutations in plant materials such as seeds, embryogenic callus, protoplasts and pollen (Settles, 2020). Then, selection for mutant may be performed in T0 plants based on the genomic sequence, whereby most mutant lines may be discarded if they do not present the interesting mutation. Additionally, selection mutant may also be performed in the first generation (T1) based on the genomic sequence, whereby most mutant lines may be discarded if they do not present the interesting mutation, and/or based on phenotype, whereby most mutant lines may be discarded if they do not present the interesting agronomic trait (e.g. heat-stress-tolerance of pollen grains). The mutation and the related agronomic traits are generally confirmed in the second and third generations mainly through phenotypic stability and/or sequencing. Finally, only the mutant lines with desirable traits and sequence are selected as a new variety or as a parent line for crossbreeding.

Applications of the methods disclosed herein include engineering changes in pollen grains heat-stress-tolerance, resulting in pollen grains with improved viability, such as greater pollen grains fertility, lower pollen grains abortion and/or lower pollen grains death, as compared to pollen grains produced by a wild-type plant or a control plant.

The techniques for transforming a plant are well known and described in the technical and scientific literature. These techniques include transformation of plant cells by injection or microinjection (Griesbach et al., 1987), electroporation of DNA (Fromm et al., 1985 ; Wan and Lemaux et al., 1994), biolistics (Klein et al., 1987), silicon carbide fiber whisker technology (Kaeppler et al, 1992), viral vector mediated approaches (Gelvin et al., 2005)) and particle bombardment (Sood et al., 2011), fusion of cells or protoplasts (Willmitzer, L., 2008), insertion of T-DNA using *Agrobacterium tumefaciens* (Fraley et al., 1986 ; Fromm et al., Biotechnology 8 (1990), 833-844) or *Agrobacterium rhizogenes* (Cho et al.,(2000) or other bacterial hosts (Brootghaerts et al., 2005) particularly using floral dip (Clough and Bent 1998; Zale et al. 2009).

In some embodiments, the E-Box motif of the PIF4 promoter is inactivated by introducing one or more mutations in the nucleotide sequence GACGTC, as disclosed above, by any methods described hereabove.

In some other embodiments, the E-Box motif of the PIF4 promoter is inactivated by introducing one or more mutations in the nucleotide sequence GACGTC by genome editing. In a particular aspect, the E-Box motif in the PIF4 promoter is inactivated by introducing one or more mutations in the nucleotide sequence ACGT of the nucleotide sequence GACGTC, as detailed above, by genome editing, preferably by a CRISPR-Cas system.

The Clustered Regularly Interspaced Shorts Palindromic Repeats (CRISPR)-Cas system is originally a bacterial defence system against foreign DNA. This system rests essentially on the association of a nuclease protein and a guide RNA (gRNA or sgRNA) responsible for the specificity of the cleavage site. It can be used to create DNA double-strand breaks (DSBs) at the sites targeted by the CRISPR/Cas system. This system has been used for targeted engineering of the genome in eukaryotic cells, such as plant cells (Shan Q et al., 2013).

In one embodiment, the CRISPR/Cas construct comprises a polynucleotide encoding a Cas endonuclease molecule, a polynucleotide encoding nuclear localization signal and at least one heterologous regulatory sequence operably linked to gRNA, wherein the gRNA is targeted to the genomic region containing the E-Box motif of GACGTC.

Several Cas endonuclease molecules (or hereafter "Cas protein") are known in the art, such as Cas9, Cas12 or Cas13.

The Cas domain is the domain of the fusion protein that can interact with the guide RNA and to target the E-Box of the PIF4 promoter in the genome. The Cas domain can consist of a Cas protein, wild-type or modified, or a fragment of this protein capable of interacting with the guide RNA. The Cas protein can notably be modified in order to modulate its enzymatic activity. The Cas protein can also be truncated to remove the protein domains not essential to the functions of the fusion protein, in particular the Cas protein domains that are not necessary to interact with the guide RNA.

For example, the Cas9 protein or fragment thereof can be obtained from any known Cas9 protein (Makarova et al., 2008). Exemplary Cas9 proteins that can be used in the present invention include, but are not limited to, the Cas9 proteins from *Streptococcus pyogenes, Streptococcus thermophilus, Streptococcus sp., Nocardiopsis dassonvillei, Streptomyces pristinaespiralis, Streptomyces viridochromogenes, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicellulosiruptor bescii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsonii, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus,* or *Acaryochloris marina.* Other Cas9 proteins that can be used in the present invention are also described in the article by Makarova et al. (Makarova et al., 2008). Preferably, the Cas9 domain comprises, or consists of, the Cas9 protein from *Streptococcus pyogenes* (NCBI entry number: WP_010922251.1) or a fragment thereof capable of interacting with the guide RNA.

The Cas endonuclease molecule can be introduced directly into a cell by any method known in the art, for example, but not limited to transient introduction methods, transfection and/or topical application.

In a particular aspect, the methods of the invention as disclosed above comprises:
a) introducing into the plant a nucleic acid comprising a guide RNA which targets sequences upstream and downstream the E-Box motif of the PIF4 promoter having a nucleotide sequence of GACGTC;
b) introducing into the plant a Cas endonuclease molecule that induces a double strand break at or near the upstream and downstream sequence of the E-Box motif; and
c) optionally recovering the seeds/plants in which the E-Box motif of GACGTC has been inactivated, such as recovering the seeds/plants in which the E-Box motif of GACGTC has been deleted.

In a particular embodiment, the guide RNA is a single guide or a dual guide RNA.

In one embodiment, the Cas endonuclease and the gRNA, preferably the dual gRNA are comprised in the same vector. The genetic transformation with a vector comprising the CRISPR/Cas system and the guide RNA can be achieved by any means, such as Floral Dip based transformation, biolistic transformation or electroporation of the designed construct. It can also be achieved by biolistic delivery of CRISPR/Cas ribonucleoproteins and in vitro gRNA transcripts.

Preferably, the introducing steps comprise delivering into the plant cell a T-DNA containing a nucleic acid sequence encoding the Cas endonuclease and a nucleic acid sequence encoding the dual guide RNA, and wherein the delivering of the T-DNA is via Agrobacterium as described hereabove.

In one embodiment, the guide RNA targets sequences in the promoter of a gene coding for PIF4, more specifically sequences flanking the E-Box motif as described herein. Preferably, the dual guide RNA targets sequences upstream and downstream of the E-Box motif of GACGTC. The dual guide RNA may also target sequences overlapping the E-Box motif of GACGTC.

In one embodiment, a dual guide RNA is used to perform the inactivation of the E-Box of GACGTC. Such dual guide comprises a first gRNA and a second gRNA.

Particularly, the first guide RNA is complementary to a sequence of 15 bp to 80 bp upstream of the E-Box motif of GACGTC. Preferably, the sequence to be targeted by the first guide RNA is within the 100, 90, 80, 70, 60, 50, 40, 30 or 20 nucleotides upstream of the first nucleotide of the E-Box motif of GACGTC. The sequence to be targeted by the first guide RNA may overlap the E-Box motif of GACGTC. Specific sequences of a first guide RNA that can be used to inactivate the E-box of the PIF4 promoter of *Solanum lycopersicum* are disclosed in the experimental part, such as in Table 1.

In one embodiment, the second guide RNA is complementary to a sequence of 15 bp to 80 bp downstream of the E-Box motif GACGTC. Preferably, the sequence to be targeted by the second guide RNA is within the 100, 90, 80, 70, 60, 50, 40, 30 or 20 nucleotides downstream of the last nucleotide of the E-Box motif. The sequence to be targeted by the second guide RNA may overlap the E-Box motif of GACGTC. Specific sequences of a second guide RNA that can be used to inactivate the E-box of the PIF4 promoter of *Solanum lycopersicum* are disclosed in the experimental part, such as in Table 1.

Other genome-editing techniques such as designer zinc fingers (ZFN), transcription activator-like effectors (TALEs or TALEN), or homing meganucleases are also available for producing targeted genome mutations.

Particularly, zinc finger is another type of DNA binding domain that can be used for introducing mutations into the target DNA. Zinc finger nucleases and transcription activator-like effector nucleases are artificial fusion proteins comprising an engineered DNA-binding domain fused to the nonspecific nuclease domain of the restriction enzyme Fok1 (Radek Jankele and Petr Svoboda, 2014; N J Palpant and D Dudzinski, 2013).

Alternatively, Transcription Activator-Like Effector Nucleases (TALENs) are artificial restriction enzymes generated by fusing the TAL effector DNA binding domain to a DNA enzyme domain. TAL proteins are produced by bacteria and include a highly conserved 33-34 amino acid DNA binding domain sequence (WO2014127287; US20140087426).

The PIF4 promoter in the plant may have two E-Box motifs, such as the E-Box motif having the nucleotide sequence of GACGTC and a E-Box motif having the nucleotide sequence of CACGTT.

In some embodiments, the PIF4 promoter has the sequence SEQ ID NO: 5, preferably the plant belongs to the species *Solanum lycopersicum* and PIF4 promoter has the sequence SEQ ID NO: 5.

In some embodiments, in particular for the species *Solanum lycopersicum,* the E-box motif having a nucleotide sequence of GACGTC is located at -600 base pairs (bp) to -700 bp from the start codon of the PIF4 gene, preferably at -625 bp to -675 bp from the start codon of the PIF4 gene, such as -676 to -670.

In some embodiments, a E-Box motif of the PIF4 promoter in the plant having a nucleotide sequence of CACGTT is not inactivated. This means that the plant producing the heat-stress-tolerant pollen grain has a nucleotide sequence of CACGTT in the PIF4 promoter. The nucleotide sequence CACGTT corresponds to a wild-type E-Box motif in the PIF4 promoter. In other words, the E-Box motif of the PIF4 promoter having the nucleotide sequence of GACGTC is the sole E-Box motif that is inactivated in the PIF4 promoter.

In some embodiments, the PIF4 promoter in the plant in which the E-Box motif of GACGTC is inactivated comprises the nucleotide sequence SEQ ID NO: 2.

In some embodiments, the plant belongs to the spermatophyte clade.

In some embodiments, the plant belongs to the angiosperm clade or gymnosperm clade.

In some embodiments, the plant belongs to a class selected from the group consisting of *Pinopsida, Cycadopsida, Ginkgoopsida,* and *Gnetopsida.*

In some embodiments, the plant belongs to a class selected from the group consisting of *Magnoliopsida or Liliopsida, preferably Magnoliopsida.*

In some embodiments, the plant belongs to an order selected from the group consisting of *Brassicales, Caryophyllales, Fabales, Lamiales, Malvales, Rosales, Sapindales,* and *Solanales,* preferably *Solanales.*

In some embodiments, the plant belongs to a family selected from the group consisting of *Convolvulaceae, Cuscutaceae, Hydroleaceae, Solanopsidaceae,* and *Solanaceae,* preferably *Solanaceae.*

In some embodiments, the plant belongs to a genus selected from the group consisting of *Cycadopsida, Ginkgoopsida, Gnetopsida, Pinopsida,* and *Solanum,* preferably *Solanum.*

In some embodiments, the plant belongs to a species selected from the group consisting of *Solanum aethiopicum, Solanum demissum, Solanum dulcamara, Solanum melongena, Solanum muricatum, Solanum nigrum, Solanum pseudocapsicum, Solanum quitosense, Solanum tuberosum,* and *Solanum lycopersicum,* preferably *Solanum lycopersicum.*

In a preferred embodiment, the plant belongs to the *Solanaceae* family, preferably the plant belongs to the *Solanum* genus, more preferably the plant belongs to the species *Solanum lycopersicum.*

Examples of plants of interest are, for example, the crops. Crops according to the invention includes cereal crops, such as wheat, rice, barley, maize, oat, sorghum, rye, onion, leek, millet, yam, buckwheat, turf grass, Italian rye grass, sugarcane or Festuca species; biofuel and bioenergy crops such as rape/canola, linseed, lupine and willow, poplar, poplar hybrids, Miscanthus or gymnosperms, such as loblolly pine; crops for silage (maize), grazing or fodder (grasses, clover, alfalfa), fibers (e.g., cotton, flax), building materials (e.g., pine, oak), pulping (e.g., poplar), feeder stocks for the chemical industry (e.g., acid oil seed rape, linseed),and ornamental crops such as snapdragon, petunia, roses, violets, Begonias, chrysanthemums and geraniums. Preferably, the plant may be selected from the group consisting of lettuce, sunflower, Arabidopsis, broccoli, spinach, watermelon, squash, cabbage, tomato, camelina, potato, yam, capsicum, tobacco, cotton, okra, apple, rose, strawberry, alfalfa, bean, soybean, field (fava) bean, pea, lentil, peanut, chickpea, apricots, pears, peach, grape vine or citrus.

### Heat-stress-tolerant pollen grain

The present invention also provides a heat-stress-tolerant pollen grain produced by a plant in which a E-Box motif of the PIF4 promoter having a nucleotide sequence of GACGTC is inactivated.

The embodiments disclosed above, for the methods of the invention, apply to the heat-stress-tolerant pollen grain of the invention.

For example, in a specific embodiment the plant belongs to the species *Solanum lycopersicum.* Therefore, the present invention provides a heat-stress-tolerant pollen grain produced by a plant belonging to the species *Solanum lycopersicum* in which a E-Box motif of the PIF4 promoter having a nucleotide sequence of GACGTC is inactivated.

In some embodiments, the heat-stress-tolerant pollen grain is produced by the method of the invention. Therefore, the heat-stress-tolerant pollen grain may be produced by a method comprising the steps of: (i) providing a plant in which a E-Box motif of the PIF4 promoter having a nucleotide sequence of GACGTC is inactivated; (ii) culturing the plant under appropriate conditions to produce pollen grains, thereby obtaining heat-stress-tolerant pollen grains.

### EXAMPLES

### Example 1:

### Materials and methods

### Plant material and growth conditions

*Solanum lycopersicum L. cv.* Micro-Tom and M82 were used for all experiments performed. Plants were cultured in a growth chamber under temperatures of 18 °C at night (8 hours) and 26°C during the day (16 hours), and 40-60% relative humidity. Plants were grown in 8cmX8cm pots, using PROVEEN soil. The plants were watered every day with a mix of HAKAPHOS CALCIDIC (1,5g/L) and BASAFER PLUS (150 mg/L). Light was supplied with VALOYA type L35 - NS12 LED tubes with a total light intensity of 309 µmol/m²/s.

### Hypocotyl elongation test under heat stress conditions

For heat stress experiments, T1 Micro-Tom seeds of SlfPIF4_MT_8, SIPIF4_MT_2, SIPIF4_MT_14 and SIPIF4_MT_3 homozygous lines were sown on MS ¼ and germinated under white light for 5 days. "SI" means "*Solanum lycopersicum".* "MT" means "MicroTom".

Then, one plate for each genotype was kept under normal temperature and photoperiod (18h/6h - 25°C/18°C) and one plate was incubated 7 days in mild heat stress (18h/6h - 30°C/30°C). After one week of incubation, hypocotyls were measured and 3 plants per genotype and per condition were transferred to soil and cultured under normal conditions (18h/6h - 26°C/18°C). After the appearance of the first flower truss, 3 plants per genotype were transferred in heat stress conditions for 7 days (18h/6h - 38°C/28°C).

### Promoter Analysis and guide design

Gene sequence data can be found in the Sol Genomics Database (https://solgenomics.net/) under the following accession numbers: SIPIF4 (Solyc07g043580). Based on the gene information, 1500 base pairs (bp) downstream the start codon were isolated from the NCBI database (MicroTom Genome PRJNA553986 & M82 PRJEB6302 respectively) and assimilated to PIF4 promoter (see Figure 1). This sequence was analysed for the presence of putative E-Box motif containing the core sequence "ACGT". Two of them were isolated for putative E-Box motif and are referenced as follows:
E-Box motif 1: GACGTC.
E-Box motif 2: CACGTT.

2 pair of guides flanking each motive were designed using CRISPOR tool (http://crispor.tefor.net/) with the following criteria:
- 5' T enriched PAM sequence: TTTC or TTTG;
- Minimal of targets (0 100% match);
- 1 forward and one reverse guide;
- Less than 80 base pairs of motif distance.

The following Table 1 discloses the guides that were designed:

| | |
|---|---|
| SIPIF4_g1_fwd (SEQ ID NO: 16) | AATCTACTCCTTTGAAACCAT |
| SIPIF4_g1_Rev (SEQ ID NO: 17) | ATGGTTTCAAAGGAGTAGATT |
| SIPIF4_g2_fwd (SEQ ID NO: 18) | AAAATTACGATCTGATATAAA |
| SIPIF4_g2_Rev (SEQ ID NO: 19) | TTTATATCAGATCGTAATTTT |
| SIPIF4_g3_fwd (SEQ ID NO: 20) | CTGTATCCAAAAAAATAATTC |
| SIPIF4_g3_Rev (SEQ ID NO: 21) | GAATTATTTTTTTGGATACAG |
| SIPIF4_g4_fwd (SEQ ID NO: 22) | GAATTGGACACAATAGTGTAA |
| SIPIF4_g4_Rev (SEQ ID NO: 23) | TTACACTATTGTGTCCAATTC |

### Plasmid constructions and plant transformation

Plasmids were built using Plantik's plasmid bank. Briefly, individual 4 guides were cloned using GoldenGate cloning (bbs1 Fast digest, Thermo Fisher) in level 1 plasmid (PL1.1 for g1, PL1.2 for g2, PL1.3 for g3 and PL1.4 for g4) containing a U6 promoter driving the expression of HH-Ribozyme, Lb-crRNA, HDV-Rib1. The level 1 constructs were assembled by GoldenGate cloning (BsaI Fast digest, ThermoFisher) into the corresponding level 2 vector (PL2.4). Finally, the guide cassette was cloned into an expression vector (pEXP3_Kana_Cas12f) using Gateway cloning (Gateway^{™} LR Clonase^{™} Enzyme mix, Thermo Fisher), containing left border (LB) and right border (RB) of the T-DNA, framing the cassette insertion site, Cas12f nuclease and a kanamycin resistance gene for selection. For M82, guide cassettes containing g1/g2 and g3/g4 were separately cloned in expression vectors. All constructs were sequenced before additional steps.

The construct was introduced into *Agrobacterium tumefaciens* strain GV3101 (https://goldbio.com/) using heat shock treatment according to the manufacture instructions. For the tomato transformation, LB medium + appropriate antibiotics were inoculated with *Agrobacterium* precultures and grown overnight until the O.D.600 reached [0.8-1.0]. The cultures were then centrifuged 15 minutes at 3500 g and the supernatant was discarded. The pellet was resuspended in LB + 200 µM acetosyringone (Thermofiher, Ref 102410025) + Thiamine (Duchefa, Ref T0614) 0.9 mg/mL and left at 28°C, 15 minutes, shaking 200 rpm to remove the antibiotics. The tubes were centrifuged for 15 minutes at 3500 g and the supernatant was discarded. Finally, the pellet was resuspended in KCMS + Acetosyringone 200µM + Thiamine 0.9 mg/mL to a final O.D.600 of 0.08 for transformation. Tomato transformation was carried out from cotyledon fragments by agroinfection. Micro Tom, M82, TORRY and TO-6242 tomato cotyledons were harvested under axenic conditions from 7-day-old seedlings sown on MS1/4 medium, cut into 3 explants, and cultured on solid KCMS medium for 48 h in the dark. Explants were then immersed for 30 min in KCMS solution + bacteria transformed with the plasmid of interest. After removing excess bacterial culture on sterile absorbent paper, explants were co-cultured on solid KCMS medium for 48 h in the dark. Explants were then rinsed 5 times with water supplemented with Tween20 0.05% (v/v) and cultured on 2Z selection medium + Kanamycin 150 µg/mL (Duchefa, Ref. K012) + Ticarcillin and clavulanic acid (GoldBio, Ref. T-104-2) at 300µg/mL. Explants were transplanted onto a new medium every week. Seedlings formed from callus were transplanted into pots containing ENR medium + Ticarcillin and clavulanic acid, 300µg/mL + kanamycin at 150 µg/mL for rooting. Induced CRISPR mutations were then monitored by PCR and sequencing before rooted plants were transferred to the growth chamber.

The following Table 2 summarizes the mediums that are used during the above protocol.

| | Solid KCMS | Liquid KCMS | 2Z | Rooting Medium |
|---|---|---|---|---|
| MS (Basal salt, Duchefa, M0221) | - | - | - | 2.2 g |
| MS (including vitamins, Duchefa, M0222) | 4.4 g | 4.4 g | 4.4 g | - |
| Sucrose | 20 g | 20 g | 30 g | 10 g |
| KH₂PO₄ | 200 g | 200 g | 200 g | - |
| pH | 5.8 | 5.7 | 5.8 | 5.8 |
| Agar | 8g | - | 4 g | 7 g |
| Thiamine | 0.9 mg/mL | 0.9 mg/mL | 0.9 mg/mL | - |
| Acetophenone | 200 µM | 200 µM | - | - |
| 2.4D | 200 µg/L | - | - | - |
| Kinetin | 100 µg/L | - | - | - |
| Nitsch Vitamins | - | - | 1X | 1X |
| Zeatin Ribosid | - | - | 2 mg/L | - |
| AIA | - | - | - | 100 µg/L |

The following mutants were identified in MicroTom, M82, TORRY, TO-6242 lines and kept for further analysis:

| **Tomato Genotypes** | **Mutant name** | **N° of E-Box deleted** |
|---|---|---|
| Microtom | SIPIF4_MT_2 | None |
| | SIPIF4_MT_3 | None |
| | SIPIF4_MT_8 | E-Box motif 1 (core motif) |
| | SIPIF4_MT_14 | E-Box motif 2 + Partial E-Box motif 1 |
| M82 | SIPIF4_M82_2 | E-Box motif 1 (core motif) |
| TORRY | SIPIF4_TORRY_4 | Partial E-Box motif 1 (core motif) |
| TO-6242 | SIPIF4_TO-6242_5 | Hetherozygous |

### DNA extraction and plant genotyping

Genomic DNA extraction was performed from young leaves (approx. 30mg) frozen in liquid nitrogen and ground using a pestle. Once ground, samples were centrifuged 1min at 5000g (4°C) then 250µL/tube of µPrep buffer was added (µPrep buffer: 25 mL S1 + 25 mL S2+ 10 mL S3, 0.2 g sodium bisulfite). Tubes were homogenised then placed in the water bath at 65°C for 1 hour, homogenising regularly by inversion. After one hour, 225µL of chloroform/isoamyl alcohol (24:1 v/v) were added and the whole was homogenised by manual inversion, then centrifuged for 10 minutes at 5000g at 20°C. The aqueous phases were collected in new tubes, then 250µL of 100% cold isopropanol was added and homogenised by inversion until the DNA has precipitated. Once precipitated, the tubes were centrifuged for 10 minutes at 5500 rpm at 4°C, the isopropanol was removed, and the pellets were rinsed with 250µL of 70% ethanol. Finally, the tubes were centrifuged for 10 minutes at 5000g at 4°C, the ethanol was removed by inverting the tubes and the pellets were speedvac dried for 15 minutes at 65°C. The pellet was finally resuspended in 50µL of mQ water and stored at -20°C.

The following Table 3 discloses the composition of the S1, S2 and S3 mediums.

| **S1** | |
|---|---|
| Sorbitol | 0.35 M |
| Tris-base | 0.1 M |
| EDTA | 5 mM |
| pH (with HCl) | 7.5 |

| **S2** | |
|---|---|
| Tris | 0.2 M |
| EDTA | 0.05 M |
| NaCl | 2 M |
| CTAB | 2% (W/V) |

| **S3** | |
|---|---|
| Sarkosyl | 5% (W/V) |

Extracted DNA was analysed by PCR and sequencing using the primers disclosed in the following Table 4:

| | |
|---|---|
| PIF4_p_Fwd (SEQ ID NO: 24) | CATCTGAAAAGTGAATCTATCATTGACA |
| PIF4_p_Rev (SEQ ID NO: 25) | GGCTAAAACTACTGCATATCAAGAAAAA |

### Pollen viability and germination assays

Fresh pollen from 3 independent WT plants and 2 independent plants from SIPIF4_MT_8 mutant was collected from mature stamens and put under heat stress conditions during 2 hours at 37°C. Pollen grains viability was then determined by Alexander staining (Alexander M, 1969). Briefly pollen grains were incubated 30 minutes at room temperature with 20µL of ready to use Alexander staining solution. The content of the tubes was then placed on a slide and viewed using a light microscope.

Pollen grains were collected from freshly dehisced anthers, and the stamens were submerged in 5mL of BK medium [1.62mM H3BO3, 1.25mM Ca(NO₃)₂, 4H₂O, 2.97mM KNO₃ and 1.65mM MgSO₄, 7H₂O] containing 10% (w/v) sucrose (Brewbaker and Kwak, 1963). Pollen grains were suspended in the BK medium by vortex, and the stamens were removed with tweezers. Tomato pollen tubes were grown in the dark, without agitation at five different temperatures 25°C. Observations were performed after 4 hours of growth.

For M82, pollen grains from tomato flowers at anthesis stage were sprayed on the top of pollen germination medium (18% sucrose, 0.01% Boric acid, 1mM CaCl₂, 1mM Ca(NO₃)₂, 1mM MgSO₄, 0.5% agar; pH=7). After 16h of incubation at 37°C in the dark, the preparation was observed using a binocular magnifier. The pollen germination rate expressed as a percentage was determined by dividing the number of germinated pollens corresponding to those emitting a pollen tube by the total number of pollen grains.

### Results

### Generation of allelic series in SIPIF4 promoter mutant population

From the entire mutant population, 18 plants were selected for presenting deletions in SIPIF4 promoter in homozygous or heterozygous state. Figure 2 represents the SIPIF4 promoter nucleotide sequences of 4 homozygous mutants having different types of deletions obtained in the population: SIPIF4_MT_3 presents intact E-Box motifs but deletions ranging from 9 to 13 in 3' of guides 1, 3 and 4. SIPIF4_MT_14 presents a large deletion of 384 base pairs, leading to a complete deletion of E-Box motif 2, and a partial deletion of E-Box motif 1. SIPIF4_MT_8 (having a PIF4 promoter comprising SEQ ID NO: 2) presents a deletion of 244 base pairs (SEQ ID NO: 1) leading to a complete deletion of the E-Box motif 1 and intact E-Box motif 2. Finally, the mutant SIPIF4_MT_2 has intact E-Box motifs but presents a deletion of 86 base pairs between the two E-Box motifs. The 18 plants were all self-pollinated for future characterisation.

### Fruit pericarp thickness was affected by the E-Box motif deletion

In normal temperature conditions, mutations in SIPIF4 promoter were associated with a range of phenotypes regarding pericarp thickness and fruit size. As an example, SIPIF4_MT_2 and SIPIF4_MT_14 present a slight reduction in fruit diameter compared to the WT whereas SIPIF4_MT_3 and 8 did not show any variation at the fruit size level (Figure 3-B). At the pericarp level both mutant SIPIF4_MT_8 and SIPIF4_MT_14 showed an increase in pericarp thickness, with SIPIF4_MT_8 having the most pronounced phenotype (Figure 3-A and 3-C).

### SIPIF4 promoters mutants showed a decrease in heat stress sensitivity

High temperature is known to induce hypocotyl elongation due to an increase in cell elongation mediated by elevated level of auxin (Gary et al, 1998). In order to evaluate the implication of the E-Box motif in SIPIF4 promoter in this mechanism, seeds from SIPIF4_MT_8 line were germinated on MS 1/4 and exposed to elevated temperature (30°C) for 7 days. WT plants showed a classical thermomorphogenesis phenotype translated by hypocotyl elongation going from 1.69 cm (+/-0.99) at RT to 3.43 cm (+/-0.73) under heat stress conditions. On the contrary, SIPIF4_MT_8 line did not show any significant difference in hypocotyl elongation under heat stress and normal temperature (1.73 +/- 0.97 at RT and 2.11 +/- 0.86) (Figure 4).

These results showed that the inactivation of E-Box motif 1 from SIPIF4 promoter decreased heat stress sensitivity of the associated mutants at the seedling stage.

### E-Box motif deletion in SIPIF4 promoter increased pollen viability and fruit setting under heat stress

To evaluate the fitness of the pollen grain after heat stress mimicking heat waves, fresh pollen from 3 independent wild type (WT) plants and two independents SIPIF4_MT_8 mutants was collected from mature stamen and exposed to high temperature (37°C) for two hours. Viability test using Alexander staining revealed that the deletion of E-Box motif 1 in mutant 8 significantly increased pollen viability from 15% compared to the WT (Figure 5A, 5B and 5C). The wild type was a plant which did not comprise inactivating a E-Box motif 1 of the Phytochrome-Interacting Factor 4 (PIF4) promoter.

In addition, a germination test after 4 hours at 37°C showed that germination efficiency of SIPIF4_MT_8 was not affected after heat stress whereas germination was decreased from 61% to 26% in WT plants. WT flowers formed under elevated temperature conditions (38°C day 16h/28°C night 8h) for 7 days, developed abnormal flower with the stigmas being visibly protruded from the anther cone that was unsoldered as described in *Pan et al., 2018* (Figure 5D). However, SIPIF4_MT_8 mutant deleted E-Box motif 1 developed normal flowers in the same conditions (Figure 5E).

These results demonstrate that the inactivation of E-Box motif 1 from SIPIF4 promoter increased heat-stress-tolerance of pollen grains.

### E-Box motif is conserved across tomato genotypes

Commercial TORRY (https://www.syngentavegetables.com/es-es/product/seed/tomate/torry) & TO-6242 (https://www.syngenta.co.in/product/seed/tomato/6242) and non-commercial varieties (M82) were sequenced on 1000 base pairs upstream the start codon of SIPIF4 coding sequence and aligned on Micro-Tom sequence to analyse the conservation of E-Box n°1 motif and associated g3/g4 guides. As shown in Figure 6, the 6 base pairs of the E-Box motif and g3/g4 showed a perfect conservation between genotypes, showing that the technology developed for Micro-Tom can be extended to other varieties of commercial interest. To do so, cotyledons from WT M82, TO-6242 and TORRY lines were transformed with a CRISPR plasmid carrying guide g3g4 through Agrobacterium mediated transformation.

After regeneration, the T0 plants were sequenced for screening of the desired mutation. Mutant SIPIF4_M82_2 was identified with a deletion of 11 base pairs around g3, including 3 of the 4 base pairs of the E-Box 1 core motive and a second deletion of 6 base pairs overlapping g4 (Figure 7). Accordingly, homozygous plants for TO-6242 were obtained and presented a shorter deletion of 8 base pairs around g3, including 1 of the 4 base pairs of the E-box core motive. Finally, only heterozygous plants were obtained for TORRY and therefore need one more generation of self-fertilization to be analyzed.

### SIPIF4 has a conserved role in pollen viability in other tomato species

Pollen germination tests under heat stress conditions were conducted by collecting fresh pollen from WT and mutant SIPIF4_M82_2 on plates containing solid germination medium and incubating them for 2 hours at 37°C. After that, germinated pollen grains for each genotype were counted under a binocular magnifier. Under heat stress conditions, pollen grains collected from SIPIF4_M82_2 presented a germination efficiency 40% higher than the WT in the same conditions (Figure 8), showing a conservation of the impact of the inactivation of E-Box motif 1 in SIPIF4 promoter on thermotolerance across tomato genotypes.

### Sequence Listing

| SEQ ID NO: | Description | Sequence |
|---|---|---|
| 1 | Deleted sequence that inactivates a E-Box | TCATATAAATTTATATCAGATCGTAATTTTCAAAACATGCCATATG TATCGATAGGAGAAAATTAAAAAATAATAAGTTATAAATTAAACT CATAAAATTTAAATTTTAAATGCCGGTGGCCTATACGGAGACGCT ATAATTGACAGCTAAAGCCACTGGTATTTGCTGTATCCAAAAAAA TAATTCAGACGTCATTTTCTCTGTCATTAATCTTATTACACTATTG TGTCCAATTCCAAAAAT |
| 2 | PIF4 promoter in which SEQ ID NO: 1 is deleted (SIPIF4_MT_8) | AATTGACTTAGTTATAACTATTTAAAAAAAACACTAATTCAATCAT AATATATTAGTAATTGACTTCATACTTGGTTACAAACATTAAAATA AAAAAACCAGTTCAATCATAATATAATAATTGACATCAATATTCTT GTCTCCTTAATCAACATCATTAGTGTTCCTTGAACACAATAATTAA AAATTATGTACTACAAGTGAAATATACATAAATAATGATTTAAAAT AATAGTGAAAATGAGGATAAGTGCAAACGTGGCAAAAGAATTTAG CAGTATTTGATCCACAAGGTGCAAAGGGTCCATAGGAATAGAAAA GAAGATTTCATACAGCTAGGCTGCACGTATCATTTTTTCCTAAAT TCACTTGATTTATTTTCCTTTCTAGTTAAAAAAAAATGACACTTTT TTATATTTAATATTTTTTCTATTTTAATTCATTGTTTGATCTATTT TTATTTTTAATCAATTTAAAAAAGAATCATTATATTCTTTTTTTAG CAACTTTATAATTTCAATCTACTCCTTTGAAAATCTTCTTACCTTT CTTACACGTTGTGTTAAATCAAAATAGGTCACTCAATTTAAAACG AAAGAATCATATTCTTAATGAAATGATTTATAGGTCACTCACATTT CCGGTCTGTTTCCTTCTTATTATTCTTGAATAAGTGAAACAACGG CCACAGAGTTTTTTTCCCTGACTTGGTTTGTTTTCCCATTATAAA ATTTTGGTGTTTCTTCTTTTTCTGAACCTAAAAGGTTTGATTTTTT TATTCTTCTTTGCTTCATTTCTTTTCTTAGTCATATTATGTTGATT TTAGAAATGGGGTTTAAAAAAGAGTGATTTTTTAAGCCATTCTTG ACTTGTTCTGTTTGTGTTGTGTGCAGGTTTTTGGTTATTTAACTT CTTTGTTTCCTTCTTTCTGTAAGGTATAATAAGGCAAAATCTTTCT TTCTGTTTCTGAGGGTTTTCTTGTTTGGGGGTGGGGTGGGGGAG GACAAGTAAGGAAAAGTAGAGGAACTATTGAAGTTTTGAAGAGT TATGTCATGCTTGTCTATATTTAGTTTCCTTTTTGCTCTATTTTAT ATGATCAAGAAATTCACTTTTTCTTGATATAGAAAAATCATTTTTT TTAAAACCCCCTTTTGACTATTAAGTATTGAATTTGATGATTTTG AGATTGCCTTTTACTGTGTGCCTAAACATACTGTTTTTTTTCTTG ATATGCAGTAGTTTTAGCC |
| 3 | Deleted sequence that inactivates a E-Box | TAATTCAGACG |
| 4 | PIF4 promoter in which SEQ ID | TTTTCAAAACATGCCATATGTATCGATAGGAGAAAATTAAAAAAT AATAAGTTATAAATTAAACTCATAAAATTTAAATTTTAAATGCCGG |
| | NO: 3 is deleted (SIPIF4_M82_2) | TGGCCTATACGGAGACGCTATAATTGACAGCTAAAGCCACTGGTA TTTGCTGTATCCAAAAAAATCATTTTCTCTGTCATTAATCTTATAT TGTGTCCAATTCCAAAAATGATGCTCACATTTCCTGTCTGTTTCC TTCTTATTATTCTTGAATAAGTGAAACAACGGCCACAGAGTTTTT TTCCCTGACTTGGTTTGTTTTCCCATTATAAAATTTTGGTGTTTC TTCTTTTTCTGAACCTAAAAGGTTGGATTTTTTAATCCTCTTGCTT CATTTCTTTTCTTAGTCATATTATGTTGATTTTAGAAATGGGGTT TAAAAAAGAGTGATTTTTTAAGCCATTCTTGACTTGTTCTGTTTG GGTTGGGTGCAGGTTTTTGGTTATTTAACTTCTTTGTTTCCTCCT TTCTGGAAGGGTTTATAAGGCAAAATCTTTCTTTCTGTTTCTGAG GGTTTTCTTGTTTGGGGGTGGGGTGGGGGAGGACAAGTAAGGA AAAGTAGAGGAACTATTGAAGTTTTGAAGAGTTATGTCATGCTTG TCTATATTTAGTTTCCTTTTTGCTCTATTTTATATGATCAAGAAAT TCACTTTTTCTTGATATAGAAAAATCATTTTTTTTAAAACCCCCTT TTGACTATTAAGTATTGAATTTGATGATTTTGAGATTGCCTTTTA CTGTGTGCCTAAACATACTGTTTTTTTTCTTGATATGCAGTAGTT TTAGCC |
| 5 | PIF4 promoter (Solyc07g04358 0) | TAATTTCAATCTACTCCTTTGAAACCATTAAAATCTTCTTACCTTT CTTACACGTTGTGTTAAATCAAAATAGGTCACTCAATTTAAAACA AAAGAATCATATTCTTAATGAAATGATTTATAGTCATATAAATTTA TATCAGATCGTAATTTTCAAAACATGCCATATGTATCGATAGGAG AAAATTAAAAAATAATAAGTTATAAATTAAACTCATAAAATTTAAA TTTTAAATGCCGGTGGCCTATACGGAGACGCTATAATTGACAGCT AAAGCCACTGGTATTTGCTGTATCCAAAAAAATAATTCAGACGTC ATTTTCTCTGTCATTAATCTTATTACACTATTGTGTCCAATTCCAA AAATGATGCTCACATTTCCCGTCTGTTTCCTTCTTATTATTCTTGA ATAAGTGAAACAACGGCCACAGAGTTTTTTTCCCTGACTTGGTTT GTTTTCCCATTATAAAATTTTGGTGTTTCTTCTTTTTCTGAACCTA AAAGGTTTGATTTTTTTATTCTTCTTTGCTTCATTTCTTTTCTTAG TCATATTATGTTGATTTTAGAAATGGGGTTTAAAAAAGAGTGATT TTTTAAGCCATTCTTGACTTGTTCTGTTTGTGTTGTGTGCAGGTT TTTGGTTATTTAACTTCTTTGTTTCCTTCTTTCTGTAAGGTATAAT AAGGCAAAATCTTTCTTTCTGTTTCTGAGGGTTTTCTTGTTTGGG GGTGGGGTGGGGGAGGACAAGTAAGGAAAAGTAGAGGAACTATT GAAGTTTTGAAGAGTTATGTCATGCTTGTCTATATTTAGTTTCCT TTTTGCTCTATTTTATATGATCAAGAAATTCACTTTTTCTTGATAT AGAAAAATCATTTTTTTTAAAACCCCCTTTTGACTATTAAGTATTG AATTTGATGATTTTGAGATTGCCTTTTACTGTGTGCCTAAACATA CTGTTTTTTTTCTTGATATGCAGTAGTTTTAGCC |
| 6 | SIPIF4 promoter (Figure 2) | TCAATCTACTCCTTTGAAACCATTAAAATCTTCTTACCTTTCTTAC ACGTTGTGTTAAATCAAAATAGGTCACTCAATTTAAAACAAAAGA ATCATATTCTTAATGAAATGATTTATAGTCATATAAATTTATATCA GATCGTAATTTTCAAAACATGCCATATGTATCGATAGGAGAAAAT |
| | | TAAAAAATAATAAGTTATAAATTAAACTCATAAAATTTAAATTTTA AATGCCGGTGGCCTATACGGAGACGCTATAATTGACAGCTAAAGC CACTGGTATTTGCTGTATCCAAAAAAATAATTCAGACGTCATTTT CTCTGTCATTAATCTTATTACACTATTGTGTCCAATTCCAA |
| 7 | SIPIF4_MT_3 (Figure 2) | TCAATCTACTCCTTTGCTTCTTACCTTTCTTACACGTTGTGTTAAA TCAAAATAGGTCACTCAATTTAAAACGAAAGAATCATATTCTTAAT GAAATGATTTATAGTCATATAAATTTATATCAGATCGTAATTTTCA AAACATGCCATATGTATCGATAGGAGAAAATTAAAAAATAATAAG TTATAAATTAAACTCATAAAATTTAAATTTTAAATGCCGGTGGCCT ATACGGAGACGCTATAATTGACAGCTAAAGCCACTGGTATTTGCT GTATCCAAAGACGTCATTTTCTCTGTCATTAATCTTATGTGTCCA ATTCCAA |
| 8 | SIPIF4_MT_14 (Figure 2) | CACTGGTATTTGCTGTATCCAAAAAAAACGTCATTTTCTCTGTCA TATTGTGTCCAATTCCAA |
| 9 | SIPIF4_MT_8 (Figure 2) | TCAATCTACTCCTTTGAAAATCTTCTTACCTTTCTTACACGTTGTG TTAAATCAAAATAGGTCACTCAATTTAAAACGAAAGAATCATATTC TTAATGAAATGATTTATAG |
| 10 | SIPIF4_MT_2 (Figure 2) | TCAATCTACTCCTTTGAAAAATCTTCTTACCTTTCTTACACGTTGT GTTAAATCAAAATAGGTCACTCAATTTAAAACGAAAGAATCATAT TCTTAATGAAAAGTTATAAATTAAACTCATAAAATTTAAATTTTAA ATGCCGGTGGCCTATACGGAGACGCTATAATTGACAGCTAAAGCC ACTGGTATTTGCTGTATCCAAAAAAATTCAGACGTCATTTTCTCT GTCATTAATCTTTATTGTGTCCAATTCCAA |
| 11 | PIF4_MT, PIF4_M82, PIF4_BO400, PIF4_TAJ, PIF4_TORRY (Figure 6) | ATGATTTATAGTCATATAAATTTATATCAGATCGTAATTTTCAAAA CATGCCATATGTATCGATAGGAGAAAATTAAAAAATAATAAGTTA TAAATTAAACTCATAAAATTTAAATTTTAAATGCCGGTGGCCTATA CGGAGACGCTATAATTGACAGCTAAAGCCACTGGTATTTGCTGTA TCCAAAAAAATAATTCAGACGTCATTTTCTCTGTCATTAATCTTAT TACACTATTGTGTCCAATTCCAAAAATGATGCTCACATTTCCCGT CTGTTTCCTTCTTATTATTCTTGAATAAGTGAAACAACGGCCACA GAGTTTTTTTCCCTG |
| 12 | SIPIF4 (Figure 7) | TATTTGCTGTATCCAAAAAAATAATTCAGACGTCATTTTCTCTGTC ATTAATCTTATTACACTATTGTGTC |
| 13 | SIPIF4_M82_2 (Figure 7) | TATTTGCTGTATCCAAAAAAATCATTTTCTCTGTCATTAATCTTAT ATTGTGTC |
| 14 | SIPIF4_TO-6242_5 (Figure 7) | TATTTGCTGTATCCAAAAAAATCGTCATTTTCTCTGTCATTAATCT TATTATTGTGTC |
| 15 | SIPIF4_TORRY_ | TATTCGTGTATCAAAAAAATATACAAACTTTGTATTAATATTAAGA |
| | 4 (Figure 7) | T |
| 16 | SIPIF4_g1_fwd | AATCTACTCCTTTGAAACCAT |
| 17 | SIPIF4_g1_Rev | ATGGTTTCAAAGGAGTAGATT |
| 18 | SIPIF4_g2_fwd | AAAATTACGATCTGATATAAA |
| 19 | SIPIF4_g2_Rev | TTTATATCAGATCGTAATTTT |
| 20 | SIPIF4_g3_fwd | CTGTATCCAAAAAAATAATTC |
| 21 | SIPIF4_g3_Rev | GAATTATTTTTTTGGATACAG |
| 22 | SIPIF4_g4_fwd | GAATTGGACACAATAGTGTAA |
| 23 | SIPIF4_g4_Rev | TTACACTATTGTGTCCAATTC |
| 24 | PIF4_p_Fwd | CATCTGAAAAGTGAATCTATCATTGACA |
| 25 | PIF4_p_Rev | GGCTAAAACTACTGCATATCAAGAAAAA |

### REFERENCES

1. Ding, Y., Shi, Y., & Yang, S. (2020). Molecular Regulation of Plant Responses to Environmental Temperatures. Molecular Plant, 13(4), 544-564. https://doi.org/10.1016/j.molp.2020.02.004
2. Ibañez, C., Delker, C., Martinez, C., Bürstenbinder, K., Janitza, P., Lippmann, R., Ludwig, W., Sun, H., James, G. V., Klecker, M., Grossjohann, A., Schneeberger, K., Prat, S., & Quint, M. (2018). Brassinosteroids Dominate Hormonal Regulation of Plant Thermomorphogenesis via BZR1. Current Biology, 28(2), 303-310.e3. https://doi.org/10.1016/j.cub.2017.11.077
3. Kan, Y., Mu, X.-R., Gao, J., Lin, H.-X., & Lin, Y. (2023). The molecular basis of heat stress responses in plants. Molecular Plant, 16(10), 1612-1634. https://doi.org/10.1016/j.molp.2023.09.013
4. Koene, S., Shapulatov, U., van Dijk, A. D. J., & van der Krol, A. R. (2023). Transcriptional Feedback in Plant Growth and Defense by PIFs, BZR1, HY5, and MYC Transcription Factors. Plant Molecular Biology Reporter, 41(1), 59-80. https://doi.org/10.1007/s11105-022-01351-9
5. Kulkarni, S. R., Vaneechoutte, D., Van de Velde, J., & Vandepoele, K. (2018). TF2Network: Predicting transcription factor regulators and gene regulatory networks in Arabidopsis using publicly available binding site information. Nucleic Acids Research, 46(6), e31. https://doi.org/10.1093/nar/gkx1279
6. PRESSMAN, E., PEET, M. M., & PHARR, D. M. (2002). The Effect of Heat Stress on Tomato Pollen Characteristics is Associated with Changes in Carbohydrate Concentration in the Developing Anthers. Annals of Botany, 90(5), 631-636. https://doi.org/10.1093/aob/mcf240
7. Rosado, D., Trench, B., Bianchetti, R., Zuccarelli, R., Rodrigues Alves, F. R., Purgatto, E., Segal Floh, E. I., Silveira Nogueira, F. T., Freschi, L., & Rossi, M. (2019). Downregulation of PHYTOCHROME-INTERACTING FACTOR 4 Influences Plant Development and Fruit Production1. Plant Physiology, 181(3), 1360-1370. https://doi.org/10.1104/pp.19.00833
8. Alexander M (1969) Differential staining of aborted and nonaborted pollen. Biotech. Histochem. pp 117-122
9. Luo, Q. (2011). Temperature thresholds and crop production: a review. Climatic change, 109(3), 583-598
10. Gray, W. M., Östin, A., Sandberg, G., Romano, C. P., & Estelle, M. (1998). High temperature promotes auxin-mediated hypocotyl elongation in Arabidopsis. Proceedings of the National Academy of Sciences, 95(12), 7197-7202. https://doi.org/10.1073/pnas.95.12.7197
11. I. C. Karapanos, K. A. Akoumianakis, C. M. Olympios & Harold Christopher Passam (2010). Tomato pollen respiration in relation to in vitro germination and pollen tube growth under favourable and stress-inducing temperatures. Sexual Plant Reproduction, Volume 23, pages 219-224. https://doi.org/10.1007/s00497-009-0132-1
12. A. Mark Settles (2020). EMS Mutagenesis of Maize Pollen. Methods in Molecular Biology (MIMB, volume 2122) pp 25-33. https://link.springer.com/protocol/10.1007/978-1-0716-0342-0_3
13. Griesbach, R. J. (1987). Chromosome-mediated transformation via microinjection. Plant Science, 50(1), 69-77
14. Fromm, M., Taylor, L. P., & Walbot, V. (1985). Expression of genes transferred into monocot and dicot plant cells by electroporation. Proceedings of the National Academy of Sciences, 82(17), 5824-5828
15. Wan, Y., & Lemaux, P. G. (1994). Generation of large numbers of independently transformed fertile barley plants. Plant physiology, 104(1), 37-48
16. 16. Klein, T. M., Wolf, E. D., Wu, R., & Sanford, J. C. (1987). High-velocity microprojectiles for delivering nucleic acids into living cells. Nature, 327(6117), 70-73
17. Kaeppler, H. F., Somers, D. A., Rines, H. W., & Cockburn, A. F. (1992). Silicon carbide fiber-mediated stable transformation of plant cells. Theoretical and applied genetics, 84, 560-566
18. Gelvin, S. B. (2005). Viral-mediated plant transformation gets a boost. Nature biotechnology, 23(6), 684-685
19. Sood, P., Bhattacharya, A., & Sood, A. (2011). Problems and possibilities of monocot transformation. Biologia plantarum, 55(1), 1-15
20. Willmitzer, Lothar. (2008). Transgenic Plants. 10.1002/9783527620838.ch16
21. Fraley, R. T., Rogers, S. G., Horsch, R. B., & Gelvin, S. B. (1986). Genetic transformation in higher plants. Critical reviews in plant sciences, 4(1), 1-46
22. Fromm, M. E., Morrish, F., Armstrong, C., Williams, R., Thomas, J., & Klein, T. M. (1990). Inheritance and expression of chimeric genes in the progeny of transgenic maize plants. Bio/technology, 8(9), 833-839
23. Cho, H. J., Farrand, S. K., Noel, G. R., & Widholm, J. M. (2000). High-efficiency induction of soybean hairy roots and propagation of the soybean cyst nematode. Planta, 210, 195-204
24. Broothaerts, W., Mitchell, H. J., Weir, B., Kaines, S., Smith, L. M., Yang, W., ... & Jefferson, R. A. (2005). Gene transfer to plants by diverse species of bacteria. Nature, 433(7026), 629-633
25. Clough, S. J., & Bent, A. F. (1998). Floral dip: a simplified method for Agrobacterium-mediated transformation of Arabidopsis thaliana. The plant journal, 16(6), 735-743
26. Zale, J. M., A;garwal, S., Loar, S., & Steber, C. M. (2009). Evidence for stable transformation of wheat by floral dip in Agrobacterium tumefaciens. Plant cell reports, 28, 903-913
27. Shan, Q., Wang, Y., Li, J., Zhang, Y., Chen, K., Liang, Z., ... & Gao, C. (2013). Targeted genome modification of crop plants using a CRISPR-Cas system. Nature biotechnology, 31(8), 686-688
28. Jiang, W., Zhou, H., Bi, H., Fromm, M., Yang, B., & Weeks, D. P. (2013). Demonstration of CRISPR/Cas9/sgRNA-mediated targeted gene modification in Arabidopsis, tobacco, sorghum and rice. Nucleic acids research, 41(20), e188-e188
29. Makarova, K. S., Wolf, Y. I., Iranzo, J., Shmakov, S. A., Alkhnbashi, O. S., Brouns, S. J., ... & Koonin, E. V. (2020). Evolutionary classification of CRISPR-Cas systems: a burst of class 2 and derived variants. Nature Reviews Microbiology, 18(2), 67-83
30. Jankele, R., & Svoboda, P. (2014). TAL effectors: tools for DNA targeting. Briefings in functional genomics, 13(5), 409-419
31. Palpant, N. J., & Dudzinski, D. (2013). Zinc finger nucleases: looking toward translation. Gene therapy, 20(2), 121-127

## Claims

1. A method for increasing heat-stress-tolerance of pollen grains of a plant, comprising inactivating a E-Box motif of the Phytochrome-Interacting Factor 4 (PIF4) promoter in the plant, wherein said E-Box motif has a nucleotide sequence of GACGTC.

2. A method for producing heat-stress-tolerant pollen grains, said method comprising the steps of:
(i) providing a plant in which a E-Box motif of the PIF4 promoter having a nucleotide sequence of GACGTC is inactivated;
(ii) culturing the plant under appropriate conditions to produce pollen grains, thereby obtaining heat-stress-tolerant pollen grains.

3. The method according to any of claims 1 or 2, wherein the E-Box motif is inactivated by introducing one or more mutations in the nucleotide sequence GACGTC.

4. The method according to claim any of the preceding claims, wherein the E-Box motif is inactivated by introducing one or more mutations in the nucleotides sequence ACGT of the nucleotide sequence GACGTC.

5. The method according to claim any of the preceding claims, wherein the one or more mutation is selected from the group consisting of an insertion, a deletion, a substitution and combinations thereof.

6. The method according to any of the preceding claims, wherein the E-Box motif is inactivated by deleting a nucleotide sequence comprising the nucleotide sequence ACGT of the nucleotide sequence GACGTC.

7. In some embodiments, the PIF4 promoter has the sequence SEQ ID NO: 5.

8. The method according to any of the preceding claims, wherein the E-Box motif is inactivated by deleting the nucleotide sequence SEQ ID NO: 1 or SEQ ID NO: 3.

9. The method according to any of the preceding claims, wherein the PIF4 promoter in the plant has two E-Box motifs, such as the E-Box motif having the nucleotide sequence of GACGTC and a E-Box motif having the nucleotide sequence of CACGTT.

10. The method according to any of the preceding claims, wherein a E-Box motif of the PIF4 promoter in the plant having a nucleotide sequence of CACGTT is not inactivated.

11. The method according to the preceding claims, wherein the PIF4 promoter in the plant in which the E-Box motif GACGTC is inactivated comprises the nucleotide sequence SEQ ID NO: 2 or SEQ ID NO: 4.

12. The method according to any of the preceding claims, wherein the plant belongs to the spermatophyte clade, such as the angiosperm clade or gymnosperm clade.

13. The method according to any of the preceding claims, wherein the plant belongs to the *Solanaceae* family, preferably the plant belongs to the *Solanum* genus.

14. The method according to any of the preceding claims, wherein the plant belongs to the species *Solanum lycopersicum.*

15. A heat-stress-tolerant pollen grain produced by a plant in which a E-Box motif of the PIF4 promoter having a nucleotide sequence of GACGTC is inactivated.
